# EUROPÄISCHE PATENTSCHRIFT

(11) **EP 3 666 831 B1**
(45) Veröffentlichungstag und Bekanntmachung des Hinweises auf die Patenterteilung: **28.06.2023**
(21) Anmeldenummer: 19215065.4
(22) Anmeldetag: 11.12.2019
(51) Int. Cl.: C09C 1/64, A61K 8/26, A61K 8/02, A61K 8/73, A61Q 3/02, C09D 5/36

(54) **GLITTER UND DESSEN VERWENDUNG IN KOSMETISCHEN FORMULIERUNGEN, BESCHICHTUNGSMATERIALIEN UND KUNSTSTOFFEN**
GLITTER AND ITS USE IN COSMETIC FORMULATIONS, COATING MATERIALS AND PLASTICS
PAILLETTES ET LEURS UTILISATION DANS DES FORMULATIONS COSMÉTIQUES, DES MATIÈRES DE REVÊTEMENT ET DES MATIÈRES PLASTIQUES

(30) Priorität: 14.12.2018 EP 18212680
(43) Veröffentlichungstag der Anmeldung: 17.06.2020
(73) Patentinhaber: Sigmund Lindner GmbH, 95485 Warmensteinach (DE)
(72) Erfinder: PSCHIERER, Erwin, 95506 Kastl (DE)
(74) Vertreter: Isarpatent

(56) Entgegenhaltungen:
- EP-A1- 2 918 630
- EP-A1- 3 106 490
- EP-A2- 2 418 089
- Bioglitter: "Bioglitter - Biodegradable in the Natural Environment", , 28. Juni 2018 (2018-06-28), XP002789831, Gefunden im Internet: URL:https://www.discoverbioglitter.com/hrf _faq/is-bioglitter-certified-to-compostabi lity-standards-copy/ [gefunden am 2019-03-19] & Bioglitter: "What is glitter and what makes Bioglitter special?", , 28. Juni 2018 (2018-06-28), Gefunden im Internet: URL:https://www.discoverbioglitter.com/faq s/ [gefunden am 2019-03-19]
- MaterialDistrict: "BioGlitz biodegradable glitter to reduce microplastics", , 13. April 2018 (2018-04-13), XP002789832, Gefunden im Internet: URL:https://materialdistrict.com/article/b ioglitz-biodegradable-glitter/ [gefunden am 2019-03-19]

## Beschreibung

Die vorliegende Erfindung betrifft einen Glitter, umfassend eine Folie, die Cellulose umfasst, dessen Verwendung in kosmetischen Produkten, Beschichtungsmaterialien (wie z. B. Farben, Lacken und Druckfarben), und Kunststoffen, sowie kosmetische Produkte, Beschichtungsmaterialien und Kunststoffe, welche den Glitter umfassen.

### Stand der Technik

Glitter finden vielfach Anwendung zur Erzeugung eines glitzernden Oberflächeneffektes und werden unter anderem und insbesondere in kosmetischen Artikeln, Farben, Lacken, Druckfarben sowie Kunststoffen eingesetzt. Zur Herstellung derartiger Glitter werden üblicherweise Folien oder Filme aus Kunststoff verwendet, die mittels eines Schneidvorganges in einzelne, vergleichbar kleine Partikel geschnitten werden. Hauptsächlich werden derzeit Filme aus Polyethylenterephthalat für die Herstellung von Glitter verwendet. In geringerem Umfang werden Filme aus biologisch abbaubaren Filmen wie z. B. Polylactose oder Celluloseacetat für die Herstellung von Glitter verwendet. Meist sind Folien, die für die Herstellung von Glitter verwendet werden, mit Aluminium beschichtet, wodurch eine reflektierende Oberfläche entsteht und ein ausgeprägter Glitzereffekt erzeugt werden kann.

Ein beispielhaftes Verfahren zur Herstellung solcher Glitter ist in der DE 102010001971 A1 offenbart. Hierin werden Glitter offenbart, die von allen Seiten beschichtet werden.

Aus ökologischen Gesichtspunkten ist es wünschenswert, Glitter aus biologisch abbaubaren Materialien herzustellen. Bekannte biologisch abbaubare Folienmaterialien, wie z. B. Polylactose und Celluloseacetat, die für die Herstellung von biologisch abbaubaren Glitter geeignet sind, sind jedoch aufgrund mangelnder Lösemittelbeständigkeit für bestimmte Anwendungen wie z. B. Nagellack nicht geeignet. Darüber hinaus haben diese biologisch abbaubaren Glitter eine geringere Temperaturbeständigkeit als Glitter basierend auf Polyethylenterephthalat.

Da Cellulose eine hohe Abbaugeschwindigkeit unter verschiedenen Bedingungen (z. B. Kompostierung und Frischwasser) aufweist, bietet Cellulose sehr gute Voraussetzungen für die Herstellung von biologisch abbaubaren Glittern.

Unter der Bezeichnung Bioglitter Sparkle werden Glitter vermarktet, die u. a. regenerierte Cellulose enthalten, die mit weiteren Polymeren beschichtet ist. Diese Glitter sind weniger lösemittelbeständig als Polyethylenterephthalat und haben eine geringere Temperaturbeständigkeit als Polyethylenterephthalat-basierte Glitter. Entsprechende Veröffentlichungen finden sich z.B. in "Bioglitter - Biodegradable in the Natural Environment" (https://www.discloserbioglitter.com/hrf_faq/is-bioglitter-certified-tocompostability-standards-copy/) (2018-06-28) sowie "What is glitter and what makes Bioglitter special?" (https://ww.discoverbioglitter.com/faqs/)(2018-06-28)

Es sind darüber hinaus metallisierte Folien bekannt, die teilweise aus Cellulose bestehen und zusätzlich mit einem Polymer beschichtet sind, wobei sich die Metallisierung außen auf der Beschichtung befindet. Zum Beispiel werden diese Folien unter dem Markennamen Natureflex NM (Fa. Futamura Chemical Co, Ltd.) vermarktet, wobei diese Folien üblicherweise mit Barriereschichten beschichtet sind (z.B. https://www.transcendia.com/sites/default/files/datasheet_download/NM-F_datasheet.pdf). Aus der EP 2 418 089 A2 sind Glitter auf Basis von modifizierter Cellulose bekannt.

In WO2012/137014A1 werden beschichtete biologisch abbaubare Filme offenbart. Die biologisch abbaubaren Polymere, die für die Beschichtung verwendet werden, sind in üblichen Lösemitteln wie z. B. Ester und Ketonen ganz oder teilweise löslich. Die mangelnde Beständigkeit zeigt sich z.B. als Glanzverlust, Deformation der Partikel, Quellung oder Auflösen der Folie. Bei darin offenbarten Folien, die mit einem Metall beschichtet sind, kann sich die Metallschicht ganz oder teilweise von der Folie ablösen.

Es besteht ein Bedarf an Glitter, welche biologisch abbaubar sind und vergleichbare anwendungstechnische Eigenschaften besitzen wie die bekannten Glitter auf Basis von Polyethylenterephthalat. Insbesondere ist eine dauerhafte Beständigkeit gegen Lösemittel wünschenswert. Darüber hinaus ist eine möglichst hohe Temperaturbeständigkeit wünschenswert.

Der Erfindung liegt daher die Aufgabe zugrunde, biologisch abbaubare Glitter bereitzustellen, welche eine verbesserte Beständigkeit gegenüber Lösemitteln aufweisen. Weiterhin ist es Aufgabe der Erfindung, biologisch abbaubare Glitter bereitzustellen, welche eine bessere Temperaturbeständigkeit aufweisen und aufgrund Ihrer guten Verträglichkeit in Kosmetikprodukten eingesetzt werden können. Weiterhin ist es Aufgabe der Erfindung, biologisch abbaubare Glitter bereitzustellen, welche aufgrund der Lösemittelbeständigkeit und Temperaturbeständigkeit in Beschichtungsmaterialien und Kunststoffen eingesetzt werden können.

### Zusammenfassung der Erfindung

Bisher wurden für die Herstellung von biologisch abbaubaren Glitter hauptsächlich Folien auf Basis von Polylactose oder Celluloseacetat verwendet. Außerdem sind Glitter bekannt, die regenerierte Cellulose enthalten, und die mit weiteren Polymeren beschichtet sind. Es hat sich überraschend gezeigt, dass die Aufgabe der Erfindung, also Glitter mit verbesserter Lösemittelbeständigkeit, gelöst werden kann, indem bei der Herstellung von Glitter Folien auf Basis von Cellulose verwendet werden, wobei die Cellulose direkt mit einem Metall beschichtet ist oder zumindest nicht mit weiteren Polymeren beschichtet ist.

Die vorliegende Erfindung betrifft somit Glitter umfassend eine Folie auf Basis von Cellulose, wobei der Glitter mit einem Metall, vorzugsweise Aluminium, beschichtet ist, wobei die Beschichtung des Metalls direkt auf der Folie, die Cellulose umfasst, erfolgt, oder wobei die Cellulose nicht direkt mit einer Polymerschicht beschichtet ist, wobei die Folie wenigstens 70 Gew.% Cellulose umfasst, bezogen auf das Gewicht der Folie. Weiterhin betrifft die Erfindung die Verwendung des Glitter in kosmetischen Formulierungen, Beschichtungsmaterialien, und Kunststoffen, sowie kosmetische Formulierungen, Beschichtungsmaterialien und Kunststoffe mit den Glittern.

In einem ersten Aspekt ist die vorliegende Erfindung auf Glitter, umfassend eine Folie, die Cellulose umfasst, gerichtet, wobei der Glitter mit einem Metall, vorzugsweise Aluminium, beschichtet ist, wobei die Beschichtung des Metalls direkt auf der Folie, die Cellulose umfasst, erfolgt, wobei die Folie wenigstens 70 Gew.% Cellulose umfasst, bezogen auf das Gewicht der Folie.

Ein zweiter Aspekt betrifft einen Glitter, umfassend eine Folie, die Cellulose umfasst, wobei die Cellulose nicht direkt mit einer Polymerschicht beschichtet ist, wobei die Folie wenigstens 70 Gew.% Cellulose umfasst, bezogen auf das Gewicht der Folie.

Weiterhin offenbart sind die Verwendung der erfindungsgemäßen Glitter in kosmetischen Produkten sowie kosmetische Produkte, umfassend die erfindungsgemäßen Glitter.

Zudem offenbart sind die Verwendung der erfindungsgemäßen Glitter in Beschichtungsmaterialien sowie Beschichtungsmaterialien, umfassend die erfindungsgemäßen Glitter. Darüber hinaus offenbart sind die Verwendung der erfindungsgemäßen Glitter in Kunststoffen sowie Kunststoffe, umfassend die erfindungsgemäßen Glitter.

Weitere bevorzugte Ausgestaltungen der vorliegenden Erfindung ergeben sich aus den abhängigen Ansprüchen und der folgenden detaillierten Beschreibung der Erfindung.

### Beschreibung der Figuren

Die beiliegenden Zeichnungen sollen Ausführungsformen der vorliegenden Erfindung veranschaulichen und ein weiteres Verständnis dieser vermitteln. Im Zusammenhang mit der Beschreibung dienen sie der Erklärung von Konzepten und Prinzipien der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander dargestellt. Gleiche, funktionsgleiche und gleich wirkende Elemente, Merkmale und Komponenten sind in den Figuren der Zeichnungen, sofern nichts anderes ausgeführt ist, jeweils mit denselben Bezugszeichen versehen.
Figuren 1 bis 5 zeigen schematisch erfindungsgemäße Glitter.
Figuren 6 bis 9 zeigen Messwerte von GC-MS- und FT-IR-Messungen von erfindungsgemäßen Glittern bzw. Glitterfolien und solchen des Stands der Technik.

### Detaillierte Beschreibung der Erfindung

### Definitionen

So nicht anderweitig definiert haben hierin verwendete technische und wissenschaftliche Ausdrücke dieselbe Bedeutung, wie sie von einem Fachmann auf dem Fachgebiet der Erfindung gemeinhin verstanden wird.

Mengenangaben im Rahmen der vorliegenden Erfindung beziehen sich auf Gew.%, soweit nicht anderweitig angegeben oder aus dem Kontext ersichtlich ist. In der Folie, die Cellulose umfasst, ergänzen sich die Gew.%-Anteile auf 100 Gew.%.

Glitter sind kleine Partikel mit verschiedensten Formen. Insbesondere weisen sie eine Größe, beispielsweise einen maximalen Durchmesser in einer Hauptausdehnungsrichtung des Partikels, von 0,02 mm bis 7,0 mm, bevorzugt 0,050 mm bis 6,0 mm, beispielsweise 0,06 mm bis 2,0 mm, z.B. 0,1 mm bis- 0,5 mm auf, beispielsweise von 100 µm bis 400 µm, auf. Hinsichtlich der Form sind die Glitterpartikel nicht besonders beschränkt und können beispielsweise als Plättchen, Nadeln, Quader, etc. vorliegen, oder zu bestimmten Formen gestanzt sein, beispielsweise Sechsecken, Quadraten, Kreisen, Ovalen, Sternen, etc. Gemäß bestimmten Ausführungsformen sind die Glitter flach ausgeführt, beispielsweise als Plättchen mit verschiedensten Formen, z.B. auch sechseckig, rechteckig, quadratisch, sternförmig, rund, oval, etc., wobei bevorzugt die Dicke der Plättchen zwischen 4 µm und 50 µm, beispielsweise zwischen 5 µm und 45 µm, z.B. zwischen 10 µm und 35 µm, beispielshaft zwischen 14 µm und 23 µm liegen kann, und/oder die Größe, beispielsweise ein maximaler Durchmesser in einer Hauptausdehnungsrichtung der Glitter, von 0,02 mm bis 7,0 mm, bevorzugt 0,050 mm bis 6,0 mm, beispielsweise 0,06 mm bis 2,0 mm, z.B. 0,1 mm bis- 0,5 mm betragen kann.

In einem ersten Aspekt betrifft die vorliegende Erfindung Glitter, umfassend eine Folie, die Cellulose umfasst, wobei der Glitter mit einem Metall, vorzugsweise Aluminium, beschichtet ist, wobei die Beschichtung des Metalls direkt auf der Folie, die Cellulose umfasst, erfolgt, wobei die Folie wenigstens 70 Gew.% Cellulose umfasst, bezogen auf das Gewicht der Folie.

In einem zweiten Aspekt ist ein Glitter offenbart, umfassend eine Folie, die Cellulose umfasst, wobei die Cellulose nicht direkt mit einer Polymerschicht beschichtet ist, wobei die Folie wenigstens 70 Gew.% Cellulose umfasst, bezogen auf das Gewicht der Folie.

Insbesondere ist die Polymerschicht hierbei eine Schicht, welche technische Eigenschaften beeinflusst, insbesondere eine Schicht zur Verbesserung der Gleitfähigkeit, eine Barriereschicht und/oder eine Schicht zur Haftungsverbesserung, wobei entsprechende Schichten durch entsprechende Polymermaterialien erzeugt werden können, welche dem Fachmann bekannt sind. Die Polymerschicht ist hierbei bevorzugt eine Schicht, die im Wesentlichen aus mindestens einem Polymer und/oder Copolymer und/oder Mischungen davon besteht oder aus einem Polymer und/oder Copolymer und/oder Mischungen davon besteht. Hierbei ist in dieser Ausführungsform die Polymerschicht jedoch keine Lackschicht. Eine Lackschicht dient im Gegensatz zur Polymerschicht im Rahmen der vorliegenden Erfindung zur farblichen Gestaltung und enthält entsprechend gemäß bestimmten Ausführungsformen mindestens ein Pigment und/oder einen Farbstoff, wobei das Pigment und/oder der Farbstoff nicht besonders beschränkt sind, jedoch nicht polymerer Natur sind.

Im Folgenden beziehen sich die Ausführungen auf den ersten und zweiten Aspekt der vorliegenden Erfindung, soweit nicht anders angegeben oder anderweitig ersichtlich.

Die Cellulose ist erfindungsgemäß nicht besonders beschränkt. Beispielsweise kann die Folie Cellulose und/oder regenerierte Cellulose und/oder Mischungen davon umfassen. Weitere Bezeichnungen für Cellulose-basierte Folien sind Zellglas, Cellophan, Cellulosehydrat oder Pergamin. Gemäß bestimmten Ausführungsformen ist die Cellulose nicht regenerierte Cellulose.

Der Ausdruck "modifizierte Cellulose" umfasst Derivate von Cellulose, also Verbindungen, welche aus Cellulose hergestellt werden können, wie beispielsweise Cellulose-Ester, z.B. Celluloseacetat, Celluloseacetatbutyrat, Celluloseacetatpropionat; Cellulosenitrat; Cellulose-Ether, z.B. Methylcellulose, Ethylcellulose, Hydroxyethylcellulose, Hydroxypropylcellulose, Carboxymethylcellulose. Der Ausdruck "regenerierte Cellulose" betrifft Cellulose, welche im Vergleich zur natürlichen Cellulose ein verändertes Kristallgitter besitzt, wobei die hochmolekulare Struktur - bestehend aus Glucosebausteinen -unverändert bleibt. Beispielsweise ist regenerierte Cellulose durch Quell- und/oder Lösungsvorgänge aus natürlicher Cellulose herstellbar.

Die Folie umfasst wenigstens 70 Gew.% Cellulose, bevorzugt mehr als 70 Gew.%, bevorzugt mehr als 75 Gew.%, bevorzugt 80 Gew.% und mehr, noch weiter bevorzugt mehr als 80 Gew.%, und insbesondere bevorzugt mehr als 90 Gew.%, beispielsweise mehr als 95 Gew.% oder sogar mehr als 99 Gew.%, bezogen auf das Gewicht der Folie. Beispielsweise besteht die Folie aus 80 Gew.% Cellulose.

Gemäß bestimmten Ausführungsformen besteht die Folie im Wesentlichen aus Cellulose oder besteht sogar aus Cellulose, abgesehen von unvermeidbaren Verunreinigungen.

Daneben können jedoch auch weitere Zusatzstoffe und/oder ein oder mehr weitere Polymere wie beispielsweise modifizierte Cellulose, Stärke, Polymilchsäure (PLA), etc., in einer Menge von bis zu 30 Gew.%, bevorzugt weniger als 30 Gew.%, bevorzugt weniger als 20 Gew.%, und insbesondere bevorzugt weniger als 10 Gew.%, beispielsweise weniger als 5 Gew.%, oder sogar weniger als 1 Gew.%, in der Folie vorhanden sein, welche nicht besonders beschränkt sind.

Beispielsweise kann die Folie als Zusatzstoff gemäß bestimmten Ausführungsformen Glyceroltriacetat/Glycerintriacetat (Triacetin), Harnstoff und/oder Glycerin enthalten. Der Massenanteil kann hierbei bevorzugt weniger als 30 Gew.%, bevorzugt weniger als 20 Gew.%, noch weiter bevorzugt weniger als 15 Gew.%, und insbesondere bevorzugt weniger als 10 Gew.%, beispielsweise weniger als 5 Gew.%, oder sogar weniger als 1 Gew.%, betragen, bezogen auf das Gewicht der Folie. Auch kann die Folie keine Zusatzstoffe enthalten. Beispielsweise könnten auch Mischungen mehrerer der genannten Zusatzstoffe enthalten sein, beispielsweise Glycerin und Harnstoff, beispielsweise in Mengen von 8 bis 14 Gew.% Glycerin und 1 bis 5 Gew.% Harnstoff, z.B. in Mengen von 10 bis 12, beispielsweise 11, Gew.% Glycerin und 2 bis 4, z.B. 3, Gew.% Harnstoff.

Alternativ oder zusätzlich kann die Folie gemäß bestimmten Ausführungsformen Additive enthalten, welche z.B. als Antiblock- und/oder Gleithilfsmittel wirken können. Diese Antiblock und/oder Gleithilfsmittel sind nicht besonders beschränkt und können beispielsweise amorphe Kieselsäuren, kristalline Kieselsäuren, Fettsäureamide und/oder Talk umfassen. Die Zugabemenge für solche Antiblock und/oder Gleithilfsmittel kann gemäß bestimmten Ausführungsformen 0 - 2,5 Gew.%, bevorzugt 0 - 1,5 Gew.%, weiter bevorzugt 0 - 1 Gew.% betragen, bezogen auf das Gewicht der Folie. Auch kann die Folie Lösungsmittel wie Wasser umfassen, beispielsweise in einer Menge von weniger als 20 Gew.%, bevorzugt weniger als 10 Gew.%, weiter bevorzugt weniger als 8 Gew.%, noch weiter bevorzugt weniger als 7 Gew.%. Gemäß bestimmten Ausführungsformen kann die Folie Lösungsmittel wie Wasser in einer Menge von mindestens 1 Gew.%, bevorzugt wenigstens mindestens 3 Gew.%, noch weiter bevorzugt wenigstens 5 Gew.%, beispielsweise 6 Gew.%.

Alternativ oder zusätzlich kann die Folie gemäß bestimmten Ausführungsformen Pigmente und/oder Füllstoffe enthalten, welche z.B. die Farbe oder die mechanischen Eigenschaften beeinflussen können. Diese Pigmente und/oder Füllstoffe sind nicht besonders beschränkt und können beispielsweise Calciumcarbonat, Magnesiumcarbonat, Bariumsulfat und/oder Mica umfassen. Der Massenanteil kann hierbei weniger als 30 Gew.%, bevorzugt weniger als 20 Gew.%, und insbesondere bevorzugt weniger als 10 Gew.%, beispielsweise weniger als 5 Gew.%, oder sogar weniger als 1 Gew.%, betragen, bezogen auf das Gewicht der Folie. Auch kann die Folie keine Pigmente und/oder Füllstoffe enthalten.

Im ersten Aspekt der vorliegenden Erfindung ist die Folie mit einem Metall, vorzugsweise Aluminium, Silber, Gold und/oder Kupfer, bevorzugt Aluminium, beschichtet. Die Beschichtung kann hierbei gemäß bestimmten Ausführungsformen auf einer Seite, auf zwei gegenüberliegenden Seiten, auf der gesamten Folie oder in anderer Weise erfolgen. Die mit Metall beschichteten Folien weisen bevorzugt eine optische Dichte von mehr als 1,0 auf, weiter bevorzugt mehr als 1,5, noch weiter bevorzugt mehr als 2,0, noch weiter bevorzugt mehr als 2,5. Die Messung der optischen Dichte kann hierbei beispielsweise mit einem Densitometer erfolgen.

Gemäß bestimmten Ausführungsformen besteht der Glitter aus der Folie, die Cellulose umfasst, wobei der Glitter mit einem Metall, vorzugsweise Aluminium, beschichtet ist, wobei die Beschichtung des Metalls direkt auf der Folie, die Cellulose umfasst, erfolgt.

Die Folie und/oder eine Metallschicht, beispielsweise eine Aluminiumschicht des ersten Aspekts können gemäß bestimmten Ausführungsformen mit einer Beschichtung auf Basis eines Polysaccharids, bevorzugt eines linearen Polysaccharids, weiter bevorzugt auf Basis von Pullulan und/oder von modifizierter Cellulose, noch bevorzugt auf Cellulosenitrat-Basis, Celluloseacetatbutyrat-Basis, Celluloseacetatpropionat-Basis und/oder Celluloseacetat-Basis und/oder Pullulan-Basis, und/oder auf Polyvinylalkohol-Basis und/oder Schellackbasis-Basis und/oder Polyurethan-Basis beschichtet sein. Auch hier kann die Beschichtung auf einer Seite, zwei gegenüberliegenden Seiten, auf der gesamten Metallschicht, beispielsweise Aluminiumschicht, der Folie und der Metallschicht, beispielsweise Aluminiumschicht, oder auf andere Weise vorhanden sein. Bevorzugt ist eine Beschichtung mit Pullulan und/oder Celluloseacetat. Gemäß bestimmten Ausführungsformen ist die Beschichtung auf der Folie und der Metallschicht.

In der Beschichtung ist das Polysaccharid nicht besonders beschränkt, ist jedoch bevorzugt ein lineares Polysaccharid, welches ebenso nicht beschränkt ist. Eine Beschichtung auf Basis eines Polysaccharids umfasst Beschichtungen mit dem Polysaccharid und/oder Derivaten des Polysaccharids, welche nicht besonders beschränkt sind. Eine Beschichtung auf Basis eines linearen Polysaccharids umfasst Beschichtungen mit dem linearen Polysaccharid und/oder Derivaten des linearen Polysaccharids, welche nicht besonders beschränkt sind. Beschichtungen auf Basis von Pullulan umfassen entsprechend Beschichtungen mit Pullulan und/oder Derivaten davon, welche nicht besonders beschränkt sind. Beschichtungen auf Basis von Cellulose umfassen Beschichtungen mit Cellulose und/oder Derviaten von Cellulose, welche nicht besonders beschränkt sind. Beschichtungen auf Basis von modifizierter Cellulose umfassen Beschichtungen mit modifizierter Cellulose. Entsprechend umfassen Beschichtungen auf Cellulosenitrat-Basis, Celluloseacetatbutyrat-Basis, Celluloseacetatpropionat-Basis und/oder Celluloseacetat-Basis Beschichtungen aus Cellulosenitrat, Celluloseacetatbutyrat, Celluloseacetatpropionat und/oder Celluloseacetat und/oder Derivaten derselben. Derviate der Beschichtungen umfassen beispielsweise Copolymere der entsprechenden Polymere und/oder Beschichtungen, in denen weitere Bestandteile umfasst sind, beispielsweise mit weniger als 50, 40, 30, 20, 10 oder 5 Gew.%, bezogen auf die Beschichtung. Die weiteren Bestandteile können solche umfassen, welche üblicherweise in Beschichtungen vorhanden sind. Bevorzugt sind jedoch im Wesentlichen keine, also weniger als 5, 4, 3, 2 oder 1 Gew.%, oder keine weiteren Bestandteile in der Beschichtung umfasst.

Gemäß bestimmten Ausführungsformen besteht der Glitter aus der Folie, die Cellulose umfasst, wobei der Glitter mit einem Metall, vorzugsweise Aluminium, beschichtet ist, wobei die Beschichtung des Metalls direkt auf der Folie, die Cellulose umfasst, erfolgt, und wobei die Folie, die Cellulose umfasst, und/oder die Metallschicht, bevorzugt die Folie, die Cellulose umfasst, und die Metallschicht,
mit einer Beschichtung auf Basis eines Polysaccharids, bevorzugt eines linearen Polysaccharids, weiter bevorzugt auf Basis von Pullulan und/oder von modifizierter Cellulose, noch bevorzugt auf Cellulosenitrat-Basis, Celluloseacetatbutyrat-Basis, Celluloseacetatpropionat-Basis und/oder Celluloseacetat-Basis und/oder Pullulanbasis, und/oder auf Polyvinylalkohol-Basis und/oder Schellackbasis-Basis und/oder Polyurethan-Basis, beschichtet ist. Bevorzugt ist eine Beschichtung umfassend Pullulan und/oder Celluloseacetat. Gemäß bestimmten Ausführungsformen ist die Beschichtung auf der Folie und der Metallschicht.

Auch in der Beschichtung können neben den genannten Materialien noch weitere Bestandteile wie etwa Pigmente und/oder Farbstoffe und/oder Lösungsmittel wie Wasser vorhanden sein.

Insbesondere bevorzugt sind Beschichtungen auf Basis von modifizierter Cellulose, bevorzugt auf Cellulosenitrat-Basis, Celluloseacetatbutyrat-Basis, Celluloseacetatpropionat-Basis und/oder Celluloseacetat-Basis, insbesondere Celluloseacetat-Basis, und/oder auf Basis von Pullulan.

Darüber hinaus kann der erfindungsgemäße Glitter auch weitere farb- und/oder effektgebende Schichten umfassen, wie sie dem Fachmann bekannt sind und die ein Fachmann geeignet beschichten kann, beispielsweise aus der Gasphase oder aus Flüssigkeit/Lösung. Beispielsweise kann der erfindungsgemäße Glitter mit einer Lackschicht beschichtet sein. Die Lackschicht ist hierbei nicht besonders beschränkt, und enthält gemäß bestimmten Ausführungsformen mindestens ein Pigment und/oder einen Farbstoff, welche nicht besonders beschränkt sind, bevorzugt jedoch nicht Polymere sind.

Gemäß bestimmten Ausführungsformen weist die Folie der erfindungsgemäßen Glitter eine Dicke von 5 µm oder mehr, bevorzugt von mehr als 10 µm, weiter bevorzugt von mehr als 13 µm, und/oder eine Dicke von 50 µm oder weniger, beispielsweise weniger als 40 µm, bevorzugt 36 µm oder weniger, auf.

Gemäß bestimmten Ausführungsformen kann die Folie zudem transparent oder im Wesentlichen transparent, beispielsweise mit einer Transmissivität bzw. einem Transmissionsgrad gegenüber Licht im sichtbaren Bereich von 380 bis 780 nm, von 70%, oder 80%, oder 90% oder mehr, sein. Gemäß bestimmten Ausführungsformen weist die Folie also eine Transmissivität gegenüber Licht im Wellenlängenbereich von 380 bis 780 nm von mindestens 70%, bevorzugt mindestens 80%, weiter bevorzugt mindestens 90%, auf. Die Transmissivität kann hier auf geeignete Weise bestimmt werden, z.B. mit einem geeigneten optischen Spektrometer.

Die Folie kann gemäß weiteren Ausführungsformen gefärbt oder ungefärbt sein, ist gemäß bestimmten Ausführungsformen jedoch ungefärbt. Zudem kann in bestimmten Ausführungsformen auf die Beschichtung mit Metall, bevorzugt Aluminium, oder die Folie und die Metallbeschichtung, oder die Folie im zweiten Aspekt, eine farbgebende Schicht aufgebracht werden.

Gemäß bestimmten Ausführungsformen kann die Folie in einem erfindungsgemäßen Glitter auch mit einer Hologramm-Prägung hergestellt werden, wie sie bei herkömmlichen Glitter bekannt ist, beispielsweise aus der US 5,810,957 oder EP2 163 381 A bekannt ist, wobei auf die beiden Dokumente hinsichtlich der Holgramm-Prägung Bezug genommen wird. Überraschenderweise lässt sich die Holgramm-Prägung auch auf der Folie umfassend Cellulose realisieren. Zur Herstellung können hierbei gängige Verfahren verwendet werden, wie das sogenannte Soft-Embossing und das Hard-Embossing.

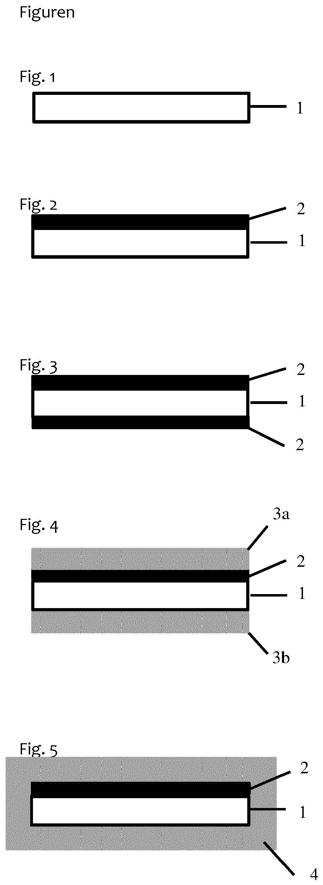

Nachfolgend wird die Erfindung näher erläutert anhand beispielhafter Ausführungsformen erfindungsgemäßer Glitter (insbesondere des ersten Aspekts in Figuren 2 bis 5), welche in Figuren 1 bis 5 gezeigt sind. Die Figuren zeigen hierbei schematisch Schnittansichten durch Glitter mit einem erfindungsgemäßen Aufbau.

Erfindungsgemäße Glitter umfassen hierbei beispielsweise Glitterpartikel mit einer Größe von 100 µm bis 200 µm und einer Dicke von 19 µm bis 30 µm, sind jedoch nicht hierauf beschränkt. Gemäß einer ersten beispielhaften Ausführungsform des zweiten Aspekts, die in Fig. 1 dargestellt ist, können transparente, im Wesentlichen aus Cellulose bestehenden Partikel 1 als Folie der erfindungsgemäßen Glitter durch Schneiden einer Cellulose-Folie gewonnen werden. Hierbei ist auf der Cellulose-Folie keine weitere Polymerschicht vorhanden.

Gemäß einer Ausführungsform des ersten Aspekts, die in Fig. 2 dargestellt ist können die im Wesentlichen aus Cellulose bestehenden Partikel 1 einseitig mit einer Metallschicht, beispielsweise einer Aluminiumschicht 2, beschichtet sein. Eine Beschichtung aus Aluminium kann hierbei vorzugsweise unter Vakuum aufgedampft werden.

Gemäß einer weiteren Ausführungsform des ersten Aspekts, die in Fig. 3 dargestellt ist können die im Wesentlichen aus Cellulose bestehenden Partikel 1 auf zwei gegenüberliegenden Seiten mit einer Metallschicht, beispielsweise einer Aluminiumschicht 2, beschichtet sein. Eine Beschichtung aus Aluminium kann hierbei vorzugsweise unter Vakuum aufgedampft werden.

Darüber hinaus können die einseitig mit Aluminium 2 beschichteten, im Wesentlichen aus Cellulose bestehenden Partikel 1 des ersten Aspekts in einer weiteren Ausführungsform, wie in Fig. 4 gezeigt, zusätzlich auf zwei gegenüberliegenden Seiten gleichmäßig mit Schichten 3a, 3b aus beispielsweise Celluloseacetat beschichtet sein.

Alternativ können die einseitig mit Aluminium 2 beschichteten, im Wesentlichen aus Cellulose bestehenden Partikel 1 des ersten Aspekts in einer weiteren Ausführungsform, wie in Fig. 5 gezeigt, auf allen Seiten gleichmäßig mit einer Schicht 4 aus beispielsweise Celluloseacetat beschichtet sein.

In den Figuren 1 bis 5 kann die im Wesentlichen aus Cellulose bestehende Folie gemäß alternativen Ausgestaltungen auch gefärbt sein. In den Figuren 4 bis 5 können zudem die Schichten aus Celluloseacetat 3a, 3b und/oder 4 auf den im Wesentlichen aus Cellulose bestehenden Partikeln gemäß alternativen Ausgestaltungen auch gefärbt sein.

Das Verfahren zur Herstellung der erfindungsgemäßen Glitter ist nicht besonders beschränkt. Eine Folie umfassend Cellulose kann hierbei geeignet zu Partikeln geeigneter Größe geschnitten und ggf. auf übliche Weise mit Metall, Polymer, etc. beschichtet werden. Hierbei können sich eine geringe Zugfestigkeit und geringe Gleitfähigkeit, beispielsweise insbesondere von Cellulose-Folien, auf die Verarbeitbarkeit bei der Glitterherstellung auswirken. Bei der Glitterherstellung werden die Folien typischerweise "von Rolle" verarbeitet. Dabei wirken in den Verarbeitungsmaschinen (z.B. Folienbeschichtung und/oder Glitterschneidmaschine) üblicherweise große Zugkräfte auf die Folien. Bei der Verarbeitung ist es daher erforderlich, die auftretenden Zugkräfte so gering wie möglich zu halten, um ein Abreißen der Folie in den Verarbeitungsmaschinen zu vermeiden. Dies kann beispielsweise durch eine entsprechend gestaltete Bahnführung in den Verarbeitungsmaschinen und die entsprechende Auslegung der bahnführenden Transportwalzen erzielt werden, um zu große Zugkräfte auf die Folie zu vermeiden.

Das Aufbringen verschiedener Beschichtungen ist nicht besonders beschränkt und kann beispielsweise aus der Gasphase und/oder aus Lösung erfolgen.

Ein weiterer Aspekt der vorliegenden Erfindung ist auf die Verwendung von erfindungsgemäßen Glittern in kosmetischen Produkten gerichtet. Das kosmetische Produkt ist hierbei nicht besonders beschränkt. Kosmetische Produkte umfassen hierbei beispielsweise Pasten, Salben, Cremes, Emulsionen, Lösungen, Lippenstift, Lip Gloss, Mascara, Mousse, Eye Shadow, Eye Liner, Puder, gepresste Puder, loses Glitterpulver, Nagellack, Seifen, Shampoo, Sonnenschutzmittel, Lotionen, Aerosol-Sprays, usw., welche die Glitter in üblichen Mengen in den Formulierungen enthalten können.

Zudem offenbart ist ein kosmetisches Produkt, welches erfindungsgemäße Glitter umfasst. Das kosmetische Produkt ist hierbei nicht besonders beschränkt, und es kann sich beispielsweise um eine Paste, eine Salbe, eine Creme, eine Emulsion, eine Lösung, einen Lippenstift, Lip Gloss, Mascara, Mousse, Eye Shadow, Eye Liner, Puder, gepressten Puder, loses Glitterpulver, Nagellack, Seife, Shampoo, Sonnenschutzmittel, eine Lotion, ein Aerosol-Spray, usw., handeln, wobei die Glitter in üblichen Mengen im kosmetischen Produkt enthalten sein können, beispielsweise zwischen 0,01 und 75 Gew.%, z.B. zwischen 1 und 10 Gew.% bezogen auf das kosmetische Produkt, oder sogar bis zu 100 Gew.% im Falle von Puder und losem Glitterpulver. Daneben können in den kosmetischen Produkten die üblichen Bestandteile vorhanden sein, wie Trägerstoffe, Füllstoffe, Öle, Wachse, Fette, Emulgatoren, Antioxidationen, Filmbildner, Geruchs- und/oder Geschmacksstoffe, Stabilisatoren, Lösemittel, Tenside, Konservierungsmittel, Verdicker, rheologische Additive, Farbstoffe, Vitamine, Puffersubstanzen, kosmetische Wirkstoffe, hautaktive Stoffe, z.B. hautpflegenden Stoffe, UV-Filter, etc., welche alle nicht besonders beschränkt sind. Die kosmetischen Produkte können beispielsweise hydrophiler, hydrophober und/oder lipophiler Natur sein. Entsprechende Bestandteile sind beispielsweise aus der DE 102005055576 A1 bekannt, auf die beispielhaft in Bezug auf kosmetische Formulierungen zur Herstellung kosmetischer Produkte Bezug genommen wird.

Zusätzlich offenbart ist die Verwendung der erfindungsgemäßen Glitter in Beschichtungsmaterialien, wie auch in Beschichtungssystemen, vorzugsweise aus dem Bereich der Lacke, Farben, Druckfarben, Kleb- und Dichtstoffe, wobei diese hier nicht besonders beschränkt ist und die Glitter in üblichen Mengen enthalten sein können. Ein Beschichtungsmaterial ist hierbei ein flüssiges, pastöses oder auch pulverförmiges Material, das vollflächig oder partiell auf Gegenstände aufgetragen wird und durch chemische oder physikalische Vorgänge eine Schicht ausbilden kann. Für die Verwendung in Druckfarben für z. B. den Tiefdruck oder Flexodruck sind eine Vielzahl von Druckfarben und Überdrucklacke geeignet, wie sie z. B. von den Firmen Marabu, Pröll, Hartmann, Siegwerk, Rotoflex, GSB-Wahl und/oder Coates Screen vertrieben werden. Die Beschichtungsmaterialien können auf Wasserbasis oder Lösemittelbasis aufgebaut sein.

Die erfindungsgemäßen Glitter sind mit einer Vielzahl an typischen Formulierungen aus den Bereichen Farben, Lacke und Kleb- und Dichtstoffe kompatibel.

Weiterhin offenbart ist ein Beschichtungsmaterial, das den erfindungsgemäßen Glitter umfasst. Die Menge der Glitter im Beschichtungsmaterial ist hierbei nicht besonders beschränkt und kann übliche Mengen umfassen, abhängig vom jeweiligen Beschichtungsmaterial. In Beschichtungsmaterialien werden die erfindungsgemäßen Glitter beispielsweise in Konzentrationen von 0,1 Gew.% bis 70 Gew.%, bevorzugt in Konzentrationen von 0,5 Gew.% bis 50 Gew.%, besonders bevorzugt in Konzentrationen von 0,5 Gew.% bis 10 Gew.%, bezogen auf das Beschichtungsmaterial. Das Beschichtungsmaterial kann beispielsweise ein Lack, eine Farbe, eine Druckfarbe, ein Klebstoff und/oder ein Dichtstoff sein.

Zudem offenbart ist die Verwendung von erfindungsgemäßen Glittern in Kunststoffen, welche beispielsweise in Dekorations- und/oder Haushaltsgegenständen Anwendung finden können. Die Kunststoffe sind hierbei nicht besonders beschränkt und umfassen beispielsweise Thermoplaste wie z. B. Polystyrol, Polypropylen, Polyethylen, und/oder Acrylnitril-Butadien-Styrol-Copolymer, Duromere wie z. B. ungesättigte Polyesterharze, Epoxidharze, und/oder Phenolharze, und/oder Elastomere wie z. B. Butadien-Kautschuk.

Weiterhin offenbart sind Kunststoffe, welche die erfindungsgemäßen Glitter umfassen. Die Kunststoffe und die Menge der darin verwendeten Glitter sind nicht besonders beschränkt und können übliche Kunststoffe und Mengen umfassen. In Kunststoffen können die erfindungsgemäßen Glitter beispielsweise in Konzentrationen von 0,01 Gew.% bis 50 Gew.%, bevorzugt in Konzentrationen von 0,05 Gew.% bis 20 Gew.%, besonders bevorzugt in Konzentrationen von 0,5 Gew.% bis 10 Gew.% enthalten sein, bezogen auf den Kunststoff. Die Kunststoffe können hierbei beispielsweise Thermoplaste wie z. B. Polystyrol, Polypropylen, Polyethylen, und/oder Acrylnitril-Butadien-Styrol-Copolymer, Duromere wie z. B. ungesättigte Polyesterharze, Epoxidharze, und/oder Phenolharze, und/oder Elastomere wie z. B. Butadien-Kautschuk umfassen oder sein.

Die obigen Ausführungsformen, Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmalen der Erfindung. Insbesondere wird der Fachmann auch Einzelaspekte als Verbesserungen oder Ergänzungen zu der jeweiligen Grundform der vorliegenden Erfindung hinzufügen.

Die Erfindung wird im Anschluss mit Bezug auf verschiedene Beispiele davon weiter im Detail erläutert. Die Erfindung ist jedoch nicht auf diese Beispiele beschränkt.

### Beispiele

### Beispiel 1: Lösemittelbeständigkeit von Glitter

Erfindungsgemäße Glitter gemäß Figur 2 wurden durch Beschichten einer Cellulose-Folie mit einer Dicke von 14 µm bis 30 µm mit Aluminium durch direktes Aufdampfen auf einer Seite und anschließendes Schneiden auf eine Größe von 100 µm bis 200 µm hergestellt.

Die Cellulose-Folie hat folgende Zusammensetzung:
80 % Cellulose (regeneriert)
11 % Glycerin
6 % Wasser
3 % Harnstoff

Die Aluminium-Beschichtung erfolgt mittels Vakuum-Bedampfung und weist eine optische Dichte von 1,9 - 2,2 auf.

Eine Messung der Partikelgröße und Partikeldicke erfolgte für ausgewählte beispielhafte Glitterpartikel aus Beispiel 1 mittels Lichtmikroskopie (Zeiss Scope A1 mit AxioCam MRc), und ergab die in Tabelle 1 angegebenen Werte.

**Tabelle 1: Messwerte von Partikeldicke und Partikelgröße von Glitterpartikeln des Beispiels 1**

| | Partikeldicke | Partikelgröße |
|---|---|---|
| Messung 1 | 22 µm | 200 µm |
| Messung 2 | 22 µm | 209 µm |
| Messung 3 | 23 µm | 208 µm |
| Messung 4 | 21 µm | 205 µm |
| Messung 5 | 22 µm | 206 µm |

Die Messung der optischen Dichte (OD = - lg I/Iₒ mit Iₒ: Intensität des eingestrahlten Lichtes) ausgewählter beispielhafter Glitterpartikel aus Beispiel 1 erfolgte mittels Densitometer (Mcbeth TD904 Rotfilter bei 624 nm).

**Tabelle 2: Messwerte der optischen Dichte von Glitterpartikeln des Beispiels 1**

| | Optische Dichte OD |
|---|---|
| Messung 1 | 2,1 |
| Messung 2 | 2,0 |
| Messung 3 | 2,0 |
| Messung 4 | 1,9 |
| Messung 5 | 2,2 |

Zum Vergleich wurden Glitter mit Folien aus Polymichsäure (PLA, polylactic acid), Celluloseacetat, Natureflex NM (einseitig metallisierte Folie mit einem mit einer Beschichtung beschichteten Cellulosefilm der Firma Innovia Films) und Polyethylenterephthalat (PET) durch entsprechende Verfahren mit der gleichen Größe und Beschichtung hergestellt. Ebenfalls wurden als Referenzbeispiele der Glitter "Bioglitter Sparkle Silver 008 (Artikelnummer 8301/008H.FDA)" der Firma Ronald Britton und der Glitter "GU CJC98AL-008H Cellulose Silver Zodiac .008 Hex" der Firma Meadowbrook Inventions, Inc. verwendet.

Mit den verschiedenen Materialien wurde die Beständigkeit gegenüber Lösemittel durch Einbringen in reines Ethylacetat für 24 h bei 50°C getestet. Hierzu wurden die Glitter in ein Probefläschchen gegeben und mit Ethylacetat überschichtet und deren Beständigkeit beobachtet. Die Ergebnisse sind in Tabelle 3 angegeben.

**Tabelle 3: Lösemittelbeständigkeit der Glitter in Beispiel 1**

| | **Cellulose** | **Celluloseacetat** | **PLA** | **Natureflex NM** | **PET** | Bioglitter Sparkle Silver 008 | Cellulose Silver Zodiac .008 hex |
|---|---|---|---|---|---|---|---|
| Ethylacetat (24 h bei 50°C) | Keine sichtbare Veränderung | Glanzverlust | Quellen und Auflösen der Folie | Glanzverlust und Ablösung der Metallisierung | Keine sichtbare. Veränderung | Glanzverlust und Ablösung der Metallisierung | Ablösung der Metallisierung |

Die Lösemittelbeständigkeit des Cellulose-basierten Glitters ist mit der Beständigkeit von Glitter auf Basis von Polyethylenterephthalat vergleichbar. Somit zeigt sich die hervorragende Verwendbarkeit von Cellulose-Folien in erfindungsgemäßen Glittern im Vergleich zu denen des Stands der Technik.

### Beispiel 2: Temperaturbeständigkeit von Glitter

Erfindungsgemäße Glitter gemäß Figur 3 wurden durch Beschichten einer Cellulose-Folie mit einer Dicke von 23 µm bis 35 µm mit Aluminium durch Aufdampfen auf zwei gegenüberliegenden Seite und anschließendes Schneiden auf eine Größe von 200 µm bis 400 µm hergestellt.

Zum Vergleich wurden Glitter mit Folien aus Polymichsäure (PLA, polylactic acid), Celluloseacetat, und Polyethylenterephthalat (PET) durch entsprechende Verfahren mit der gleichen Größe und Beschichtung hergestellt. Ebenfalls wurde zum Vergleich wiederum der Glitter "Bioglitter Sparkle Silver 008" der Firma Ronald Britton verwendet.

Mit den verschiedenen Materialien wurde die Temperaturbeständigkeit getestet. Hierzu wurden die Glitter erwärmt und visuell beobachtet. Die maximale Temperaturbeständigkeit ist hierbei dann die Temperatur, bis zu der keine Veränderung des Glitters festgestellt werden kann. Bei höheren Temperaturen treten dann optische Veränderungen wie z.B. Glanzverlust auf. Die Ergebnisse sind in Tabelle 4 angegeben.

**Tabelle 4: Temperaturbeständigkeit der Glitter in Beispiel 2**

| | **Cellulose** | **Celluloseacetat** | **PLA** | **PET** | Bioglitter Sparkle Silver 008 |
|---|---|---|---|---|---|
| Maximale Temperaturbeständigkeit in °C | 240°C | 120°C | 60°C | 220°C | 80°C |

Die Temperaturbeständigkeit des Cellulose-basierten Glitters übertrifft die Temperaturbeständigkeit von Glitter auf Basis von Polyethylenterephthalat, wie auch der anderen Materialien.

Somit zeigt sich die hervorragende Verwendbarkeit von Cellulose-Folien in erfindungsgemäßen Glittern im Vergleich zu denen des Stands der Technik.

### Beispiel 3: Applikationsbeispiele erfindungsgemäßer Glitter

In den nachfolgenden Formulierungsbeispielen werden jeweils ein erfindungsgemäßer Glitter (Glitterbeispiel gemäß Beispiel 1) und ein dem Stand der Technik entsprechender Glitter (Vergleichsglitter bestehend aus einem Polyethylenterephthalat-Film mit Al-Beschichtung mit gleicher Größe aus Beispiel 1) vergleichend beurteilt.

Mit diesen Glittern wurden verschiedene Produkte aus den Bereichen Kosmetik, Lack, Druckfarbe und Kunststoff auf übliche Weise hergestellt, mit den Bestandteilen/Inhaltsstoffen und Anteilen (Gew.%, bezogen auf das Produkt) gemäß den jeweiligen Angaben in Tabellen 5, 6 und 7.

### Produkt 1: Nagellack

**Tabelle 5: Zusammensetzung Nagellack**

| | **Inhaltsstoff** | **%W/W** |
|---|---|---|
| **Phase A** | Ethylacetat | 31,0 |
| | Butylacetat | 23,0 |
| | Nitrocellulose | 13,0 |
| | Phthalsäure-Anhydrid/TrimellitsäureAnhydrid/Glykol Copolymer | 7,0 |
| | Isopropanol | 5,0 |
| | Dipropylenglykoldibenzoat | 5,0 |
| | n-Butanol | 5,0 |
| | Silica | 3,0 |
| | Acetyltributylcitrat | 2,0 |
| | Acrylat Copolymer | 2,0 |
| | Ethanol | 1,0 |
| **Phase B** | Glitter | 3,0 |
| | **Summe** | 100,0 |

### Herstellung:

1. Inhaltsstoffe der Phase A mischen
2. Phase B in Phase A einmischen

Prüfung der Lagerfähigkeit: Lagerung des Glitter enthaltenden Nagellackes für 7 Tage bei 50°C.

Die Cellulose-basierten Glitters haben eine vergleichbar gute Lagerfähigkeit wie Glitter auf Basis von Polyethylenterephthalat. Es ist keine optische Veränderung der Cellulose-basierten Glitter im Nagellack während der Lagerzeit erkennbar.

Somit zeigt sich die hervorragende Verwendbarkeit von Cellulose-Folien in erfindungsgemäßen Glittern im Vergleich zu denen des Stands der Technik.

### Produkt 2: Einbrennlack

**Tabelle 6: Zusammensetzung Einbrennlack**

| **Inhaltsstoff** | **%W/W** |
|---|---|
| Setal 90173 SS-50, Fa. Allnex | 50,0 |
| Setamin US 138 BB-70, Fa. Allnex | 15,0 |
| Methoxypropylacetat | 14,5 |
| Butylacetat | 14,5 |
| CAB 381-0.5, Fa. Eastman | 5,0 |
| Glitter | 1,0 |
| **Summe** | 100 |

### Herstellung:

### Inhaltsstoffe mischen

### Verarbeitung:

Lack mit Spiralrakel (100 µm) auf Aluminium-Blech applizieren. 20 Minuten bei Raumtemperatur ablüften lassen. 30 Minuten bei 160 °C einbrennen.

Die Cellulose-basierten Glitters haben eine vergleichbar gute Temperaturbeständigkeit wie Glitter auf Basis von Polyethylenterephthalat.

Somit zeigt sich die hervorragende Verwendbarkeit von Cellulose-Folien in erfindungsgemäßen Glittern im Vergleich zu denen des Stands der Technik.

### Produkt 3: Druckfarbe

**Tabelle 7: Zusammensetzung Druckfarbe**

| **Inhaltsstoff** | **%W/W** |
|---|---|
| FlexiPrint MV Verschnittlack, Fa. Flint Group | 97,0 |
| Glitter | 3,0 |
| **Summe** | 100,0 |

### Herstellung:

### Inhaltsstoffe mischen

### Verarbeitung:

Druckfarbe mit Spiralrakel (20 µm) auf Aluminium-Blech applizieren. 5 Minuten bei Raumtemperatur ablüften lassen. 15 Minuten bei 140°C trocknen.

### Produkt 4: Kunststoff

1 kg LLDPE (linear low-density polyethylen; lineares Polyethylen niederer Dichte) werden in einem Taumelmischer mit 5 g Haftmittel gleichmäßig benetzt. Dazu werden 10 g Glitter zugegeben und 2 min lang gemischt und anschließend auf einem Doppelwellen-Extruder ein Granulat hergestellt. Dieses Granulat wird auf einer Spritzgießmaschine unter üblichen Bedingungen zu Stufenplättchen mit den Maßen 4 × 6 cm verarbeitet.

Der erfindungsgemäße Glitter zeigt in den Kunststoffplättchen stark reflektierende, punktuelle Glitzereffekte.

### Beispiel 4: Temperaturbeständiger Glitter mit enger Partikelgrößenverteilung.

Es wurde ein erfindungsgemäßer Glitter gemäß Figur 3 mit folgender Zusammensetzung (Gew.%) hergestellt:
99,5 % Pergamin 40 g/m²
0,5 % Aluminium

Das Pergamin hatte die folgende Zusammensetzung in Gew.%:
88,5 % Cellulose
6,5 % Wasser
2,5 % Calciumcarbonat
2,5 % Magnesiumcarbonat

Die Aluminium-Beschichtung des Pergamin erfolgte direkt darauf mittels Vakuum-Bedampfung.

Eine Messung der Partikelgröße und Partikeldicke in Beispiel 4 erfolgte mittels Lichtmikroskopie, wie in Beispiel 1. Die Ergebnisse der Messung sind in Tabelle 8 dargestellt.

**Tabelle 8: Messwerte von Partikeldicke und Partikelgröße von Glitterpartikeln des Beispiels 4**

| | Partikeldicke | Partikelgröße |
|---|---|---|
| Messung 1 | 30 µm | 371 µm |
| Messung 2 | 31 µm | 387 µm |
| Messung 3 | 30 µm | 389 µm |
| Messung 4 | 30 µm | 382 µm |

### Beispiel 5: Lösemittelbeständiger Glitter mit Pullulan-Beschichtung

Der Glitter aus Beispiel 1 wird mittels Wirbelschichtverfahren farbig beschichtet.

Hierfür werden 1,0 kg Glitter in den Vorratsbehälter einer Wirbelschichtanlage (ProCell Lab System, Fa. Glatt) gegeben und mit 80 m³/h verwirbelt. Die Zuluft-Temperatur beträgt 50 °C. Der Farblack, bestehend aus 25 g Pullulan (CAS 9057-02-7), 150 g Wasser und 20 g Tartrazin-Gelb (CI 19140), wird mit einem Sprühdruck von 1,6 bar innerhalb von 30 Minuten auf den Glitter aufgesprüht.

Der dabei entstehende goldfarbige Glitter entspricht der schematischen Darstellung gemäß Figur 5.

Beispielhafte Dickenmessungen (gemessen wie in Beispiel 1) ergaben die folgenden Werte: 22 µm, 20 µm, 24 µm, 23 µm.

Für den hergestellten Glitter wird die Lösemittelbeständigkeit ("Ausbluten") folgendermaßen untersucht:
0,2 g Glitter werden in einem Reagenzglas vorgelegt und mit 3 ml Methylethylketon überschichtet und mit einem Stopfen verschlossen. Nach 1 Minute wird das Reagenzglas für 5 Sekunden geschüttelt und anschließend bei Raumtemperatur gelagert. Die Lösemittelbeständigkeit wird visuell anhand der Verfärbung des Methylethylketons beurteilt:
0 : keine Verfärbung = lösemittelbeständig
1: sichtbare Verfärbung = nicht lösemittelbeständig

Als Vergleichsglitter wurde der Bioglitter Sparkle Gold der Fa. Ronald verwendet.

Die Ergebnisse der Messung sind in Tabelle 9 gezeigt.

**Tabelle 9: Messwerte der Lösemittelbeständigkeit in Beispiel 5:**

| | Nach 5 Minuten | Nach 30 Minuten | Nach 5 Stunden | Nach 24 Stunden |
|---|---|---|---|---|
| Glitter des Beispiels 5 | 0 | 0 | 0 | 0 |
| Bioglitter Sparkle Gold, Fa. Ronald Britton | 1 | 1 | 1 | 1 |

### Beispiel 6: Farbig beschichteter Glitter

Der Glitter aus Beispiel 1 wird mittels Wirbelschichtverfahren farbig beschichtet.

Hierfür werden 1,0 kg Glitter in den Vorratsbehälter der Wirbelschichtanlage (ProCell Lab System, Fa. Glatt) gegeben und mit 80 m³/h verwirbelt. Die Zulufttemperatur beträgt 50 °C. Der Farblack, bestehend aus 20 g Celluloseacetat, 15 g Eisenoxidgelb und 100 g Methylethylketon, wird mit einem Sprühdruck von 1,6 bar innerhalb von 45 Minuten auf den Glitter aufgesprüht.

Der dabei entstehende Glitter entspricht der schematischen Darstellung gemäß Figur 5.

### Vergleichsbeispiel 1:

Es wurden Vergleichsversuche mit Bioglitter der Fa. Ronald Britton durchgeführt. Der Bioglitter pure der Fa. Ronald Britton enthält keine Aluminium-Beschichtung und unterscheidet sich somit von dem der vorliegenden Erfindung.

Zur Untersuchung des Schichtaufbaus wurde ein Bioglitter Sparkle der Fa. Ronald mittels FT-IR und Pyrolyse-GC/MS untersucht, um zu untersuchen, wie sich die Unterschiede in der Lösemittelbeständigkeit und Temperaturbeständigkeit erfindungsgemäßer Glitter sowie derer der Fa. Ronald erklären lassen.

Das verwendete Pyrolyse-GC/MS-System besteht aus Trace GC (Fa.Thermo Scientific) und DSQ (Fa. Thermo Scientific) mit Pyrola 2000 (Fa. Pyrolabs),
Analysenbedingungen:
- Probenmenge: ca. 0,5 mg
- Pyrolyse: 500 °C / 10 s / He
- GC: 2 min 100 °C / 15 K/min 6 280 °C / 25 min 280 °C
- Säule: Restec Q-Bond /1,5 ml/min He
- DSQ: EI+ 200 °C

### Durchführung:

Von den Proben wurden Teile (ca. 0,2 mg) entnommen bzw. abgetrennt, auf das Filament der Pyrola 2000 aufgebracht und bei ca. 500 °C / 10 s pyrolysiert. Die Pyrolyseprodukte werden mit dem Gasstrom direkt auf die GC-Säule gespült und nach der Auftrennung mittels Massenspektrometrie (Totalionenstrom-Chromatogramm) analysiert.

Die FTIR-Analyse wurde mittels ATR-Methode durchgeführt:
Golden Gate Diamant ATR-Einheit, FMIR-Einheit
Wellenzahlen: 4000 cm-1 bis 600 cm-1
Auflösung: 4 cm-1, Anzahl Scans: 16

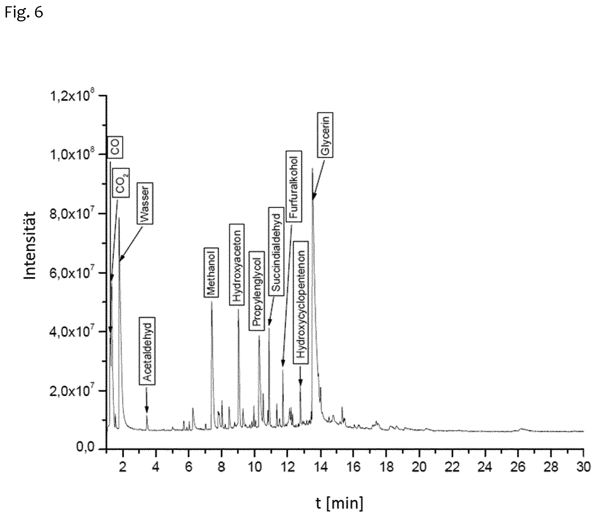

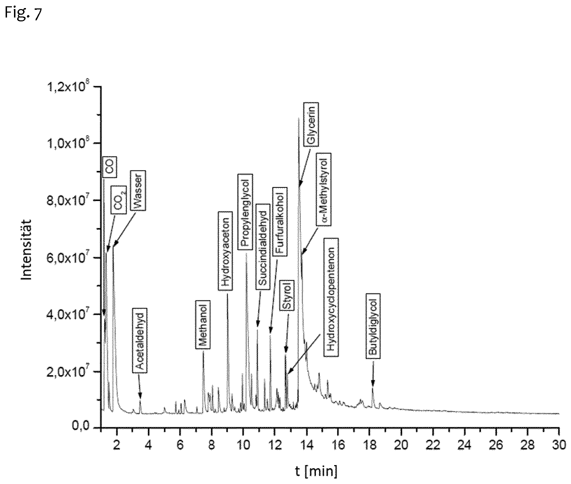

Die Untersuchung ergab, dass in dem Bioglitter Sparkle der Fa. Ronald zusätzliche Polymer-Schichten enthalten sind. Dies wird aus Figuren 6 und 7 (Pyrolyse-GC/MS) und 8 und 9 (FT-IR) ersichtlich. Figuren 6 und 7 zeigen hierbei die Ergebnisse einer Pyrolyse-GC/MS bei 500°C für 10 s mit einer Restec Q-Bond Säule. Aus den Figuren ist ersichtlich, dass Bioglitter Sparkle in Figur 7 im Gegensatz zu dem erfindungsgemäßen Glitter aus Beispiel 1, gezeigt in Figur 6, zusätzlich Styrol, Methylstyrol und Butyldiglycol enthält. Styrol und Methylstyrol weisen auf eine Sytrol/Acrylatbeschichtung hin, und Butyldiglycol auf eine Beschichtung auf Polyesterbasis.

Diese Ergebnisse decken sich mit der FTIR-Untersuchung von kommerziell erhältlichen metallisierten Cellulose-Folien (Fa. Futamura, Natureflex NM), wie sie in Beispiel 1 als Vergleichsfolien verwendet wurden.

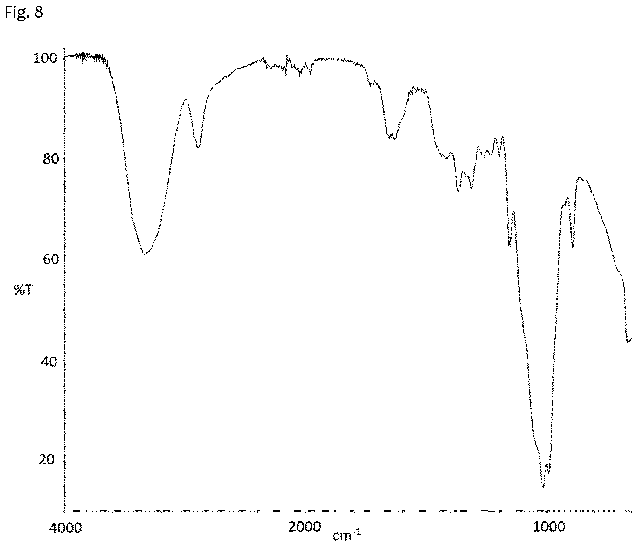

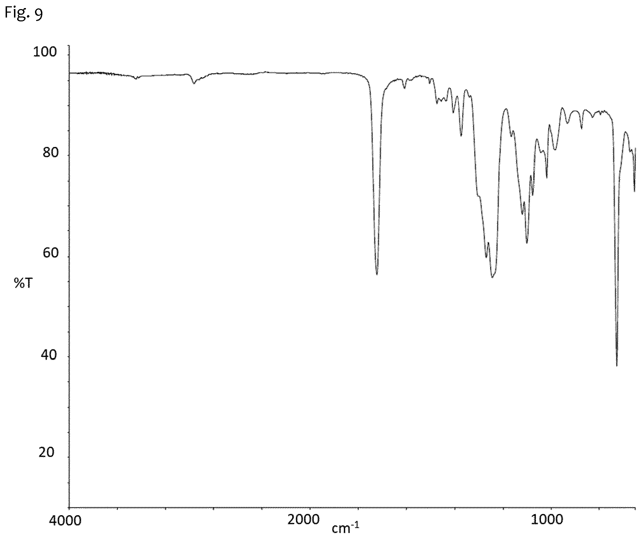

Die FT-IR-Spektren (Perkin Elmer Spektrum BX; ATR-Einheit, FMIR-Einheit, Wellenzahlen: 4000 cm-1 bis 600 cm-1, Auflösung: 4 cm-1, Anzahl Scans: 16) sind in Figuren 8 und 9 gezeigt, wobei in Figur 8 eine erfindungsgemäße Folie gemessen wurde, und in Fig. 9 die Folie Futamura, Natureflex NM. Die erfindungsgemäße Folie (Aufbau gemäß Fig. 2; 79,0 Gew.% Cellulose (regeneriert), 11 Gew.% Glycerin, 6 Gew.% Wasser, 3 Gew.% Harnstoff, 1 Gew.% Aluminium) in Fig. 8 zeigt nur Banden, die typisch für Zellglas sind. Die Folie Natureflex NM in Fig. 9 zeigt zusätzliche Banden, die auf eine Beschichtung auf Polyesterbasis hinweisen.

### Beispiel 7:

Ein weiterer beispielgemäßer Glitter wurde wie in Beispiel 5 hergestellt, wobei die Beschichtung mit Pullulan durch eine Beschichtung mit Celluloseacetat ersetzt wurde. Es ergab sich für diesen Glitter dieselbe Lösemittelbeständigkeit wie für den Glitter in Beispiel 5.

## Patentansprüche

1. Glitter, umfassend eine Folie, die Cellulose umfasst, wobei der Glitter mit einem Metall, vorzugsweise Aluminium, beschichtet ist, wobei die Beschichtung des Metalls direkt auf der Folie, die Cellulose umfasst, erfolgt, wobei die Folie wenigstens 70 Gew.% Cellulose umfasst, bezogen auf das Gewicht der Folie.

2. Glitter, umfassend eine Folie, die Cellulose umfasst, wobei die Cellulose nicht direkt mit einer Polymerschicht beschichtet ist, wobei die Folie wenigstens 70 Gew.% Cellulose umfasst, bezogen auf das Gewicht der Folie.

3. Glitter gemäß Anspruch 1, wobei die Folie mit einer Beschichtung auf Basis von modifizierter Cellulose, bevorzugt auf Cellulosenitrat-Basis, Celluloseacetatbutyrat-Basis, Celluloseacetatpropionat-Basis und/oder Celluloseacetat-Basis, und/oder auf Polyurethan-Basis, beschichtet ist.

4. Glitter gemäß Anspruch 1 oder 3, wobei eine Metallschicht, beispielsweise eine Aluminiumschicht, mit einer Beschichtung auf Basis von Cellulose oder modifizierter Cellulose, bevorzugt auf Cellulosenitrat-Basis, Celluloseacetatbutyrat-Basis, Celluloseacetatpropionat-Basis und/oder Celluloseacetat-Basis, und/oder auf Polyurethan-Basis, beschichtet ist

5. Glitter gemäß einem der Ansprüche 1, 3 oder 4, wobei die mit dem Metall beschichtete Folie des Glitters eine optische Dichte von mehr als 1,0 aufweist.

6. Glitter gemäß einem der vorigen Ansprüche, wobei die Folie aus Cellulose besteht.

7. Glitter gemäß einem der vorigen Ansprüche, wobei die Folie eine Dicke von 5 µm oder mehr aufweist, und wobei die Folie eine Dicke von 50 µm oder weniger aufweist.

8. Glitter gemäß einem der vorigen Ansprüche, wobei die Folie eine Transmissivität gegenüber Licht im Wellenlängenbereich von 380 bis 780 nm von mindestens 70% aufweist.

9. Verwendung von Glitter gemäß einem der Ansprüche 1 - 8 in kosmetischen Produkten.

10. Kosmetisches Produkt, umfassend einen Glitter gemäß einem der Ansprüche 1 - 8.

11. Verwendung von Glitter gemäß einem der Ansprüche 1 - 8 in Beschichtungsmaterialien.

12. Beschichtungsmaterial, umfassend einen Glitter gemäß einem der Ansprüche 1 - 8.

13. Verwendung von Glitter gemäß einem der Ansprüche 1 - 8 in Kunststoffen.

14. Kunststoff, umfassend einen Glitter gemäß einem der Ansprüche 1 - 8.

## Claims

1. Glitter comprising a film comprising cellulose, wherein the glitter is coated with a metal, preferably aluminium, the metal being coated directly onto the film comprising cellulose, the film comprising at least 70% by weight cellulose based on the weight of the film.

2. Glitter comprising a film comprising cellulose, wherein the cellulose is not directly coated with a polymer layer, the film comprising at least 70% by weight cellulose based on the weight of the film.

3. Glitter according to claim 1, wherein the film is coated with a coating based on modified cellulose, preferably on cellulose nitrate, cellulose acetate butyrate, cellulose acetate propionate and/or cellulose acetate, and/or based on polyurethane.

4. Glitter according to either claim 1 or claim 3, wherein a metal layer, for example an aluminium layer, is coated with a coating based on cellulose or modified cellulose, preferably on cellulose nitrate, cellulose acetate butyrate, cellulose acetate propionate and/or cellulose acetate, and/or based on polyurethane.

5. Glitter according to any of claims 1, 3 and 4, wherein the metal-coated film of the glitter has an optical density of more than 1.0.

6. Glitter according to any of the preceding claims, wherein the film consists of cellulose.

7. Glitter according to any of the preceding claims, wherein the film has a thickness of 5 µm or more and wherein the film has a thickness of 50 µm or less.

8. Glitter according to any of the preceding claims, wherein the film has a light transmissivity of at least 70% in the wavelength range from 380 to 780 nm.

9. Use of glitter according to any of claims 1-8 in cosmetic products.

10. Cosmetic product comprising a glitter according to any of claims 1-8.

11. Use of glitter according to any of claims 1-8 in coating materials.

12. Coating material comprising a glitter according to any of claims 1-8.

13. Use of glitter according to any of claims 1-8 in plastics materials.

14. Plastics material comprising a glitter according to any of claims 1-8.

## Revendications

1. Paillettes comprenant un film, qui comprend de la cellulose, lesdites paillettes étant revêtues avec un métal, de préférence de l'aluminium, le revêtement du métal ayant lieu directement sur le film qui comprend de la cellulose, le film comprenant au moins 70 % en poids de cellulose, par rapport au poids du film.

2. Paillettes comprenant un film, qui comporte de la cellulose, dans lesquelles la cellulose n'est pas directement revêtue avec une couche de polymère, dans lesquelles le film comprend au moins 70 % en poids de cellulose par rapport au poids du film.

3. Paillettes selon la revendication 1, dans lesquelles le film est recouvert avec un revêtement à base de cellulose modifiée, de préférence à base de nitrate de cellulose, à base d'acéto butyrate de cellulose, à base d'acéto propionate de cellulose, et/ou à base d'acétate de cellulose, et/ou à base de polyuréthane.

4. Paillettes selon la revendication 1 ou la revendication 3, dans lesquelles la couche de métal, par exemple une couche d'aluminium, est recouverte avec un revêtement à base de cellulose ou de cellulose modifiée, de préférence à base de nitrate de cellulose, à base d'acéto butyrate de cellulose, à base d'acéto propionate de cellulose, et/ou à base d'acétate de cellulose, et/ou à base de polyuréthane.

5. Paillettes selon l'une des revendications 1, 3 ou 4, dans lesquelles le film revêtu de métal des paillettes présente une densité optique supérieure à 1,0.

6. Paillettes selon l'une des revendications précédentes, dans lesquelles le film est constitué de cellulose.

7. Paillettes selon l'une des revendications précédentes, dans lesquelles le film présente une épaisseur égale ou supérieure à 5 µm, et dans lesquelles le film présente une épaisseur inférieure ou égale à 50 µm.

8. Paillettes selon l'une des revendications précédentes, dans lesquelles le film présente une transmissivité par rapport à la lumière dans la plage des longueurs d'onde 380 à 780 nm d'au moins 70 %.

9. Utilisation de paillettes selon l'une des revendications 1 à 8 dans des produits cosmétiques.

10. Produit cosmétique, comprenant des paillettes selon l'une des revendications 1 à 8.

11. Utilisation de paillettes selon l'une des revendications 1 à 8 dans des matériaux de revêtement.

12. Matériau de revêtement, comprenant des paillettes selon l'une des revendications 1 à 8.

13. Utilisation de paillettes selon l'une des revendications 1 à 8 dans des matières plastiques.

14. Matière plastique comprenant des paillettes selon l'une des revendications 1 à 8.
